# EUROPEAN PATENT APPLICATION

(11) **EP 2 868 267 A1**
(43) Date of publication of application: **06.05.2015**
(21) Application number: 14814651.7
(22) Date of filing: 30.06.2014
(51) Int. Cl.: A61B 5/01

(54) **BODY TEMPERATURE MEASUREMENT DEVICE, BODY TEMPERATURE MEASUREMENT SYSTEM AND BODY TEMPERATURE MEASUREMENT METHOD**

(30) Priority: 01.07.2013 JP 2013138504
(71) Applicant: Willile Mobile, Inc., Kyoto-shi, Kyoto 600-8441 (JP)
(72) Inventor: SOGO, Shinji, Kyoto-shi Kyoto 600-8441 (JP); AKAGI, Sadayuki, Kyoto-shi Kyoto 600-8441 (JP)
(74) Representative: Brookes Batchellor LLP
(86) International application number: PCT/JP2014/067473
(87) International publication number: WO 2015/002160

(57) **Abstract**

Provide a body temperature measuring apparatus 1 which can measure body temperature with high accuracy, is a non-battery type and a non-contact type, and realize improvement in accuracy in measuring body temperature with software version upgrades easily.

A body temperature measuring apparatus is comprised of a terminal apparatus 10 and a body temperature detection unit 11. The body temperature detection unit 11 comprises a power supply unit 11b for converting a voice output signal from the terminal apparatus into electricity, a detection unit 11c for detecting infrared energy from the user's skin surface as a temperature detection signal, and a conversion unit 11d for converting the temperature detection signal into a voice input signal. The terminal apparatus 10 comprises a oscillation means for oscillating a voice output signal to the body temperature detection unit 11, and a measurement means for estimating the user's body temperature corresponding to the surface temperature of which the variation is small based on the voice input signal received form the body temperature detection unit 11, an emissivity of the user's skin surface, and the temporal variation of the surface temperature calculated within a predetermined measurement time.

## Description

### TECHNICAL FIELD

The present invention relates to a body temperature measuring apparatus, a body temperature measuring system, and a body temperature measuring method.

### BACKGROUND ART

Up the present, many technologies have come into existence which measure and display body temperature data of users with the non-contact method. For example, Patent Literature 1, Patent Literature 2, Patent Literature 3, and Patent Literature 4 disclose a non-contact-type thermometer using an infrared detection element.

Also, up to the present, many technologies have come into existence which creates databases from body temperature data. For example, Patent Literature 5, Patent Literature 6, Patent Literature 7, Patent Literature 8, Patent Literature 9, and Patent Literature 10 disclose a system which creates and utilizes a database from body temperature data of individuals.

### PRIOR ART DOCUMENTS

### Patent Literature

Patent Literature 1: Japanese Unexamined Patent Application Publication No. 2011-177499
Patent Literature 2: Japanese Unexamined Patent Application Publication No. 2011-179986
Patent Literature 3: Japanese Unexamined Patent Application Publication No. 2012-34731
Patent Literature 4: Japanese Unexamined Patent Application Publication No. 2012-231309
Patent Literature 5: Japanese Unexamined Patent Application Publication No. 2001-353160
Patent Literature 6: Japanese Unexamined Patent Application Publication No. 2007-330328
Patent Literature 7: Japanese Unexamined Patent Application Publication No. 2008-264352
Patent Literature 8: Japanese Unexamined Patent Application Publication No. 2010-148689
Patent Literature 9: Japanese Unexamined Patent Application Publication No. 2011-92611
Patent Literature 10: Japanese Unexamined Patent Application Publication No. 2012-71054

### SUMMARY OF INVENTION

### Problems to Be Solved by the Invention

In recent years, the spread of terminal apparatuses such as smart phones has made it easier for users to obtain health information on the internet. As a result, health consciousness of users is on a rise. On the other hand, although body temperature is essential for health management, development of a health management system which combines a thermometer for measuring the body temperature with the terminal apparatus is insufficient. As a result, there is a need for a tool where the user can easily measure and gain data on his or her own body temperature and manage his or her own health.

In addition, applications for the terminal apparatus continue evolving on an everyday basis while these applications have immediate reactions from the public on a domestic and international level because of technological advances, including the internet. While many applications are created each and every day, if a specific function for an application works well for users, this function easily spreads across the world. Under these conditions, if a health management system which combines the thermometer with the terminal apparatus is provided to users and then body temperature data are collected through the internet from users owning the apparatus over time while correlating these data to attribute information of users, including sex, location, age and nationality, this could become enormous amount of unstructured data (big data). If this big data is distinguished between body temperature of the user and body temperatures of other users and processed properly, this could yield useful health information which wasn't possible up to now.

Accordingly, the present invention was created as a solution for the problems and aims at providing a non-battery type and non-contact type body temperature measuring apparatus, a body temperature measuring system, and a body temperature measuring method which can measure body temperature with high precision while making it possible to improve the precision of body temperature measurements through software version upgrades.

### Solution to Problem

After conducting rigorous and repeated research, the present inventors have completed a novel body temperature measuring apparatus, a novel body temperature measuring system and a novel body temperature measuring method in accordance with the present invention. That is, this novel body temperature measuring apparatus in association with this invention consists of a terminal apparatus and a body temperature detection unit which are connected through a voice input/output hole and a voice input/output terminal, and includes the following configurations. The body temperature detection unit comprises a power supply unit for converting a voice output signal from the terminal apparatus into electricity and supplying the converted electricity to its respective units of the body temperature detection unit, a detection unit for detecting infrared energy from the user's skin surface separated from a detection surface to a predetermined measurement distance as a temperature detection signal, and a conversion unit for converting the temperature detection signal into voice input signal and transmitting the converted voice input signal to the terminal apparatus. In addition, the terminal apparatus comprises a oscillation means for oscillating a voice output signal to the power supply unit of the body temperature detection unit, and a measurement means for calculating surface temperature of the skin surface based on the voice input signal received from the conversion unit and emissivity of the user's skin surface and estimating the user's body temperature corresponding to the surface temperature of which the variation is small based on the temporal variation of the surface temperature calculated within a predetermined measurement time.

Also, the present invention comprises a body temperature measuring system that connects the body temperature measuring apparatus connected in a communicable way to a server through a network, and includes the following configurations. The terminal apparatus comprises a display reception means for displaying the user's estimated body temperature and receiving a selection of a account from the user. The server comprises a management means for storing the user's body temperature in association with the user's account received from the terminal apparatus and making the terminal apparatus display the user's body temperature in association with the user's account and, the user's body temperature estimated before.

Also, the present invention is a body temperature measuring method of a body temperature measuring apparatus comprising a terminal apparatus and a body temperature detection unit that are connected through a voice input/output hole and a voice input/output terminal, and includes the following configurations. The body temperature measuring method comprises a step of oscillating a voice output signal from the terminal apparatus to the body temperature detection unit, a step of converting the voice output signal into electricity and supplying the converted electricity to its respective units of the body temperature detection unit, a step of detecting infrared energy as a temperature detection signal from the user's skin surface separated from a detection surface at a predetermined measurement distance, a step of converting the temperature detection signal into a voice input signal and transmitting the converted voice input signal to the terminal apparatus, and a step of calculating surface temperature of the skin surface based on the voice input signal received form the body temperature detection unit and the emissivity of the user's skin surface, and estimating the user's body temperature corresponding to the surface temperature of which the variation is small based on the temporal variation of the surface temperature calculated within a predetermined measurement time.

### Advantageous Effects of Invention

According to the present invention, a body temperature measuring apparatus, a body temperature measuring system and a body temperature measuring method can measure body temperature with high accuracy, a non-battery type, and a non-contact type, and allows improvement in accuracy for measuring body temperature with software version upgrades easily.

### BRIEF DESCRIPTION OF DRAWINGS

FIG. 1 is a diagrammatic illustration indicating an example of a body temperature measuring apparatus in accordance with an embodiment of the present invention.
FIG. 2A is an illustration indicating a non-contact type body temperature measurement of a body temperature measuring apparatus in accordance with an embodiment of the present invention.
FIG. 2B is an illustration indicating a contact type body temperature measurement of a body temperature measuring apparatus in accordance with an embodiment of the present invention.
FIG. 3 is an illustration indicating a relationship between measurement time and surface temperature calculated and a relationship between measurement time and variation in accordance with an embodiment of the present invention.
FIG. 4 is a diagrammatic illustration indicating an example of a body temperature measuring system in accordance with an embodiment of the present invention.
FIG. 5 is a functional block diagram of a body temperature measuring apparatus and a body temperature measuring system in accordance with an embodiment of the present invention.
FIG. 6 is a flowchart indicating execution steps in accordance with an embodiment of the present invention.
FIG. 7A is an illustration indicating an example of a body temperature measurement screen in accordance with an embodiment of the present invention.
FIG. 7B is an illustration indicating an example of a display screen of the body temperature measurement screen in accordance with an embodiment of the present invention.
FIG. 8A is an illustration indicating an example of a user temperature table in accordance with an embodiment of the present invention.
FIG. 8B is an illustration indicating an example of a display screen of the body temperature measurement screen in accordance with an embodiment of the present invention.
FIG. 9A is an illustration indicating an example of a body temperature history screen in accordance with an embodiment of the present invention.
FIG. 9B is an illustration indicating an example of the first body temperature map screen according to the present invention;
FIG. 10A is an illustration indicating an example of the second body temperature map screen in accordance with an embodiment of the present invention.
FIG. 10B is an illustration indicating an example of the third body temperature map screen in accordance with an embodiment of the present invention.
FIG. 11A is an illustration indicating an example of the fourth body temperature map screen in accordance with an embodiment of the present invention.
FIG. 11B is an illustration indicating an example of menstruation cycle screen in accordance with an embodiment of the present invention.

### DESCRIPTION OF EMBODIMENTS

The preferred embodiments of a body temperature measuring apparatus, a body temperature measuring system and a body temperature measuring method in the present invention will be explained below according to the attached drawings; thereby the present invention will be clearly understood. The embodiments below are examples materializing the present invention, and do not limit the technical scope of the present invention.

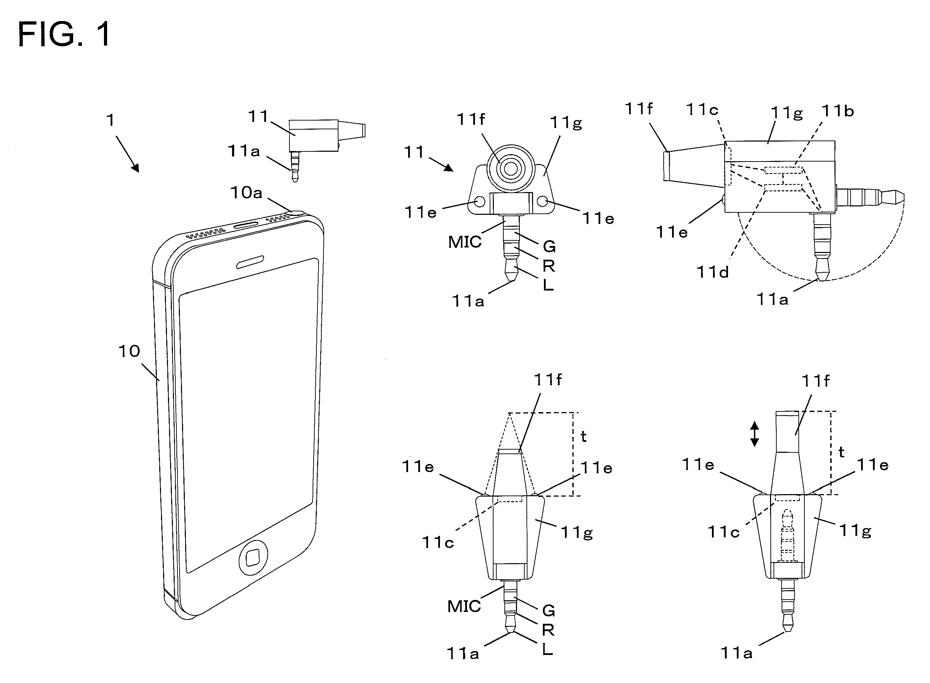

As shown in Fig. 1, a body temperature measuring apparatus 1 in accordance with the present invention comprises a terminal apparatus 10 and a body temperature detection unit 11 that are connected through a voice input/output hole 10a (jack) and a voice input/output terminal 11 a (plug). The terminal apparatus 10 is a generally used computer, a portable notebook computer, a portable terminal apparatus with a touch panel and so on. The terminal apparatus 10 comprises a display unit (output unit) for displaying screens, reception unit (input unit) for receiving a predetermined instruction input from a user, memory unit for memorizing predetermined information, and control unit for controlling its respective units.

If the terminal apparatus 10 is a computer or notebook computer, a liquid crystal display becomes the display unit, and both the keyboard and mouse becomes the reception unit. If the terminal apparatus 10 is a portable terminal apparatus with a touch panel such as a smartphone, the touch panel serves as both a display unit and reception unit. The terminal apparatus 10 is a portable terminal apparatus with a touch panel as follows.

In addition, for example, the terminal apparatus 10 includes CPU, ROM, RAM, and HDD not shown built-in, and CPU uses RAM as a working area and executes specific applications software (program) stored in ROM, HDD and so on. CPU executes the software for materializing the undermentioned means.

The body temperature detection unit 11 on the present invention is constructed to be electrically attachable/detachable to the terminal apparatus 10 through the voice input/output hole 10a and voice input/output terminal 11 a. In this way, this makes it easy to make it available to users by realizing a association with the terminal apparatus 10 and the body temperature detection unit 11 through a generally common voice input/output connection.

Here, the voice input/output terminal 11a of the body temperature detection unit 11 does not have any limitation in particular. For example, if the voice input/output terminal 11 a and the voice input/output hole 10a are a CTIA type, the voice input/output terminal 11a becomes a quadrupole plug comprising form the top end to the bottom end in an order of a voice output terminal to the left (L), a voice output terminal to the right (R), a grounding (G) terminal, and a voice input terminal (MIC), and the voice input/output hole 10a becomes a quadrupole earphone jack meeting global standards which works with the quadrupole plug. Also, if the voice input/output terminal 11 a and voice input/output hole 10a are the OMTP type, the voice input/output terminal 11 a comprises from the top end to the bottom end in an order of a voice output terminal to the left (L) and right (R), a voice input terminal (MIC), and a grounding (G) terminal, and the voice input/output hole 10a corresponds to this.

In the present invention, the body temperature detection unit 11 includes a power supply unit 11 b for converting a voice output signal from the terminal apparatus 10 into electricity and supplying the converted electricity to respective units of the body temperature detection unit 11, a (infrared rays) detection unit 11c for detecting infrared energy as a temperature detection signal (numerical value signal) from the user's skin surface separated from a detection surface to(at) a predetermined measurement distance t (a few cm), and a conversion unit 11d (conversion module) for converting the temperature detection signal into a voice input signal and transmitting the converted voice input signal to the terminal apparatus 10.

Also, the terminal apparatus 10 includes an oscillation means (software) for oscillating a voice output signal to the power supply unit 11b, and a measurement means (software) for calculating surface temperature (radiation temperature) of the skin surface based on the voice input signal received form the conversion unit 11d and an emissivity of the user's skin surface and estimating the user's body temperature corresponding to the surface temperature of which the variation is small based on the temporal variation of the surface temperature calculated within a predetermined measurement time.

Here, for example, when the voice input/output hole 10a and the voice input/output terminal 11 a are a CTIA type and if the oscillation means oscillates the voice output signal of the voice output terminal (L/R), the power supply unit 11b can generate electricity of 1000 .mu.A at approximately 1.8V with the voice output signal. In this case, when electricity shortage occurs, the power supply unit 11b is built-in a predetermined rectification circuit in advance, the oscillation means oscillates a voice output signal of approximately 20kHz, and the power supply unit 11 b can generate high voltage of approximately 4.5V by rectifying the voice output signal oscillated. Furthermore, the power supply unit 11b can generate an electricity of 300 .mu.A at approximately 1.8V by using the plug-in power of the voice input terminal (MIC). The plug-in power means that the voltage of number V is applied to the voice input terminal (MIC) of the voice input/output hole 10a in advance, and the body temperature detection unit 11 receives a power supply from the terminal apparatus 10 by the voice input terminal (MIC) of the voice input/output terminal 11a making contact with the voice input terminal (MIC) of the voice input/output hole 10a. In this way, it is possible to provide electricity to the body temperature detection unit 11 by using the voice input/output signal, and make the body temperature detection unit 11 a non-battery type and maintenance-free. In addition, the voltage value and the current value are examples of the experiment for a CTIAtype, and are changed depending on the type used for the voice input/output terminal 11a and voice input/output hole 10a. Further, a signal composition separation unit for composing the voice input/output signal and separating the voice output signal (for supplying power) and the voice input signal (temperature data use) can be equipped to include both the body temperature measurement and the power supply.

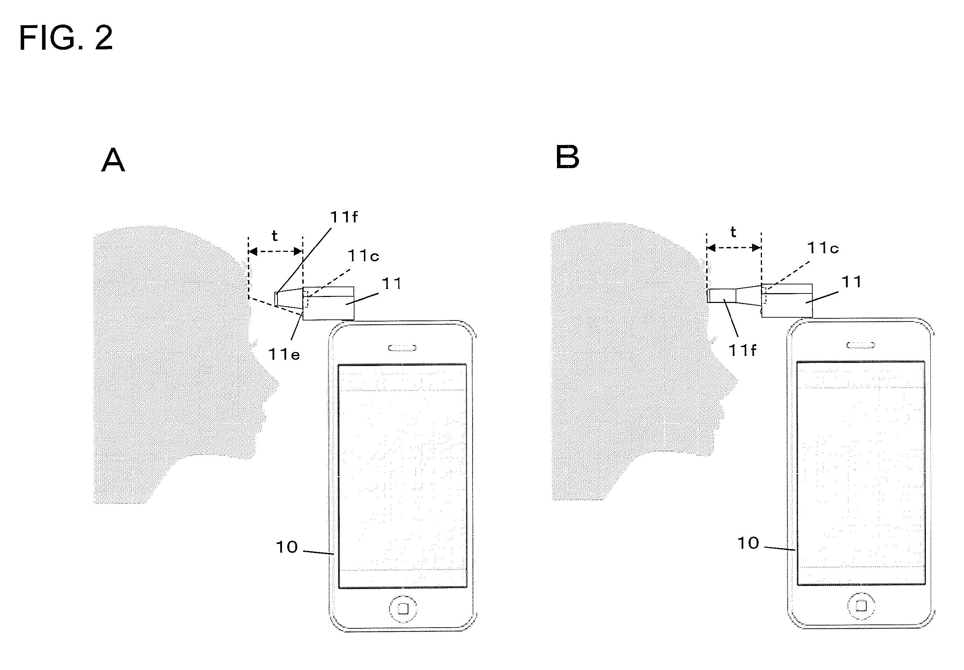

As shown in Fig. 2A, when a user keeps the measurement distance t from their own skin surface (e.g., a forehead) and holds the detection surface of the detection unit 11c, the detection unit 11c receives infrared energy emitted from the user's skin surface as a temperature detection signal. And the conversion unit 11d converts the temperature detection signal into a voice input signal, and the measurement means receives the voice input signal through the voice input terminal (MIC).

In a non-contact type temperature measurement, the value which is obtained by multiplying infrared energy by a reciprocal number of the emissivity (0.95, 0.98) becomes the surface temperature of an object emitting the infrared energy, and a tiny variation of the emissivity influences the measurement accuracy of the surface temperature greatly. For example, the measurement accuracy of the surface temperature when not considering the emissivity is several degrees whereas the measurement accuracy of the surface temperature when considering the emissivity is several hundredths of a degree to several tenths of a degree. Therefore, in the present invention, the measurement means applies a predetermined compensation formula using the emissivity of the user's skin surface to the voice input signal received, and calculates the surface temperature of the skin surface. In this way, it is possible to improve the measurement accuracy of the user's body temperature from a few degrees to several hundredths of a degree to several tenths of a degree. In addition, the emissivity is set in advance, but this can be regulated when necessary through selections and software version upgrades by the user.

Also, with the non-contact type temperature measurement, the longer the measurement time becomes, the temporal variation (change, inclination, variation per unit time of the surface temperature, a rate of increase) of the calculated surface temperature is smaller. When the measurement time is infinite, the temporal variation of the calculated surface temperature becomes approximately 0 and the surface temperature converges to a specific surface temperature. The temporal variation of the calculated surface temperature is a concept differed from a measurement error. For example, when the measurement time is short, the detection unit 11c does not correspond to the environmental temperature, the detection unit 11c cannot detect (acquire) infrared energy from the user skin surface fully, and the user's body temperature of the skin surface is unstable. Therefore, when the measurement time is short, the calculated surface temperature is usually measured lower than the specific surface temperature. On the other hand, when the measurement time is long, the detection unit 11c corresponds to the environmental temperature and the detection unit 11c can detect infrared energy from the user's skin surface fully. In addition, the user is relaxed psychologically, and the user's body temperature of the skin surface is stable. Therefore, when the measurement time is long, the calculated surface temperature converges to the specific surface temperature.

Particularly, in the non-contact type temperature measurement, depending on the environment temperature and the length of the measurement distance between the detection surface of the detection unit 11c and the user's skin surface, the infrared energy which the detection unit 11c detects is not stabilized immediately. In addition, with the user's body temperature measurement, the user's body temperature of the skin surface subtly fluctuates depending on the stress level of the user. For example, when the measurement time is short, the user's body temperature of the skin surface changes due to the fluctuation of blood flow by the measurement operation of the user, and the calculated surface temperature does not stabilize immediately. On the other hand, if the measurement time becomes long, the measurement operation of the user stops and the calculated surface temperature stabilizes.

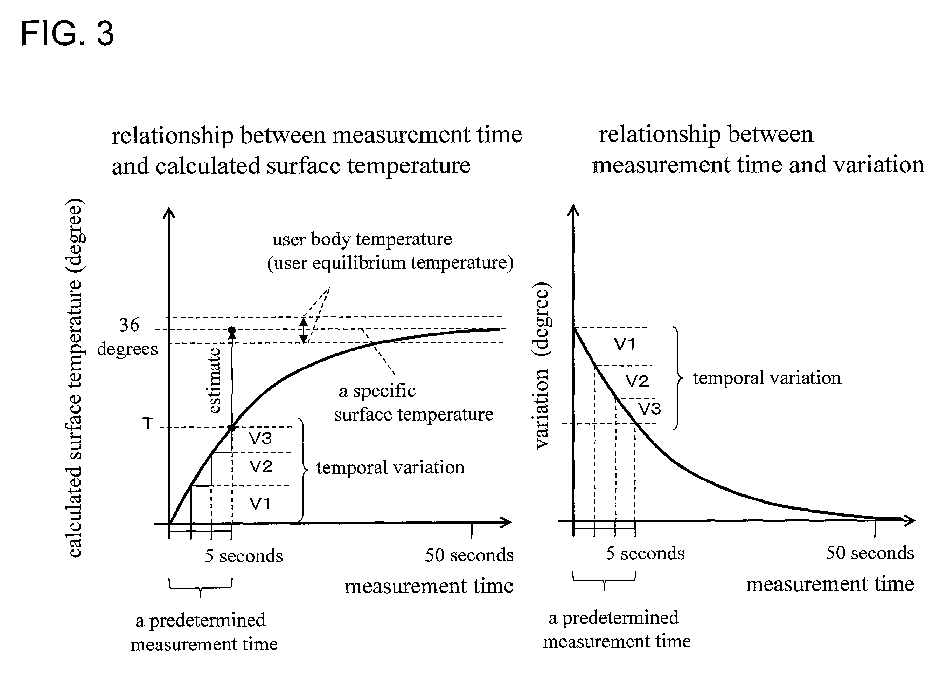

Therefore, if the final calculation of the surface temperature is used, the measurement accuracy theoretically improves. For example, as shown in Fig.3, if the measurement time is 5 seconds, the temporal variation of the calculated surface temperature is several degrees to the specific surface temperature. If the measurement time is 50 seconds, the temporal variation of the calculated surface temperature is several tenths of a degree to the specific surface temperature. Such phenomenon is seen in even a conventional contact-type thermometer (mercurial thermometer, electronic thermometer). For example, it takes about 10 minutes at the underarm (armpit) or about 5 minutes at the mouth (sublingual) to measure the user's equilibrium temperature corresponding to the specific surface temperature which converges.

Here, when the convenience of the user is considered, it is preferable that the measurement time is short. Once the temporal variation of the surface temperature calculated within a predetermined measurement time is decided, it is possible to univocally decide the specific surface temperature which converges. As shown in Fig.3, if the variation (V1+V2+V3) of the surface temperature is fixed at a predetermined measurement time (5 seconds), it is possible to estimate (back calculate) the specific surface temperature (36 degrees) which converges with the inclination of the following variations. Therefore, the measurement means calculates the temporal variation of the surface temperature within the measurement time based on the surface temperature calculated within a predetermined measurement time (e.g., 5 seconds - 30 seconds), and estimates the specific surface temperature by using a predetermined compensation formula which can be used for back calculating the specific surface temperature from the variation. Specifically, the surface temperature T right after the measurement time (5 seconds) is added to the correction value (several degrees) decided from the variation (V1+V2+V3), the specific surface temperature is back calculated. This corresponds to the compensation formula. In addition, the user's body temperature (user equilibrium temperature) means generally the user's internal body temperature. So in reality, the specific surface temperature is corrected even more depending on conditions, including environmental temperatures, and the user's final body temperature is estimated.

In addition, the specific surface temperature can be the surface temperature when the measurement time is infinite or the surface temperature when the measurement time is several minutes to several hours. In this way, even if a measurement time is the measurement (5 seconds - 30 seconds) time when the variation of the surface temperature (several degrees) is large, it is possible to measure the user's body temperature having a small variation (several tenths of a degree).

Also, by the compensation formula being changed depending on environmental temperature, age, sex, race, or the like accordingly, it is possible to improve the measurement accuracy of the user's body temperature. For example, the compensation formula is changed with a software version upgrade. In addition, for example, the user's skin surface of the forehead or the ear can be used as a user's skin surface. And, the object for temperature measurement is applicable to the surface of objects other than the human body.

Here, as shown in Fig. 1 and Fig. 2A, for example, it is preferable for the body temperature detection unit 11 to also be equipped with a pair of light emission units 11 e (LED pointer as a focus) for focusing laser beams on the measurement distance t from the detection surface. In this way, while watching a mirror, the user can adjust the position from their own skin surface to the detection surface of the body temperature detection unit 11 c until the user sees the focusing point of the laser beam on the skin surface. And it is possible to prevent the occurrence of measurement errors (variation) for each body temperature measurement.

Also, the detection unit 11c type does not have any limitation in particular while the detection unit 11c is applicable to the type with which a reception surface (emission surface of infrared energy) receiving infrared energy becomes wider when the measurement object is separated from the detection surface or the type with which the reception surface becomes narrower when the measurement object is separated from the detection surface. In addition, the measurement distance t is set at 2cm - 3cm.

Also, the body temperature detection unit 11 preferably is provided with a cylinder unit 11f for being extendable from the detection surface of the detection unit 11c to the measurement distance t. As shown in Fig. 2B, for example, the user extends the cylinder unit 11f and contacts the top of the cylinder unit 11f with their skin surface (e.g., forehead). Then, the distance from the top of the cylinder unit 11f to the detection surface of the detection unit 11c becomes the measurement distance t exactly. In this way, it is possible to prevent measurement errors for every body temperature measurement even more. In addition, if the user extends the cylinder unit 11f, the user can measure the user's body temperature with a (pseudo) contact type. And if the user shortens the cylinder unit 11f, the user can measure the user's body temperature with a non-contact type.

Here, it is preferable for the contact type body temperature measurement to be used for the body temperature measurement of a user individually, and it is preferable for the non-contact type body temperature measurement to be used for the body temperature measurements of other users (friend, family, or the like) other than the user as individuals. In this way, it is possible to prevent an outbreak of infection (secondary infection) through contact between a user and other users and spread of infection. Some of the examples of the infections that can be pointed out are diseases, including influenza, measles, MRSA (methicillin-resistant staphylococcus aureus), SARS (Severe Acute Respiratory Syndrome). This configuration is especially effective when measuring a number of users' body temperatures with a single body temperature measuring apparatus 1 in public institutions such as hospitals, clinic, and schools.

In addition, the conventional non-contact-type thermometers are in most cases is a stand type, which makes carrying inconvenient. But the body temperature detection unit 11 according to the present invention can be easily attached to and detached from the terminal apparatus 10, and is optimal for carrying around the body temperature detection unit 11.

Furthermore, body unit 11g of the body temperature detection unit 11 is rotatably supported on the bottom end of the voice input/output terminal 11a. In this way, the user can change the angle of the body temperature detection unit 11 with respect to the terminal apparatus 10 easily, and the body temperature measuring apparatus 1 as a whole can be miniaturized. In addition, regarding the dimension of the body temperature detection unit 11, the length of the body unit 11g is set within 40 mm, and the width and the height of the body unit 11g are set within 30 mm.

In this way, the body temperature detection unit 11 is equipped with a minimum number of units necessary for acquiring the user's body temperature, and eliminates the necessity of a liquid crystal display unit which is responsible for breakdowns. Therefore, it is possible to achieve a reduction in size, weight, and price for the body temperature detection unit 11, eliminate the need for maintenance, and allows it to be highly durable.. In addition, if an exterior of the body temperature detection unit 11 is composed from passive metal such as aluminum, it is possible to improve the durability even more. Furthermore, the power supply to the body temperature detection unit 11 and the measurement (conversion) of the user's body temperature are realized by execution of the specific application software of the terminal apparatus 10. Therefore, if the user installs the specific application software in the terminal apparatus 10 which doesn't need a battery, and executes this, the user can measure the user's body temperature with ease. Also, once the manufacturer manufactures the body temperature detection unit 11 and provides it to the user, the manufacturer can improve the measurement accuracy of the body temperature and the performance by providing the software and implementing software version upgrades afterward.

For example, the manufacturer first developed the first software (measurement means, algorithm) which receives a number of voice input signals in a predetermined time (1 second - 5 seconds), calculates the standard deviation (or the error) for the number of voice input signals, and estimates the body temperature mean and the error of the body temperature by using the compensation formula/correction formula. Then, new knowledge was discovered. Next, the manufacturer developed the second software which measures the user's body temperature based on the received voice input signals and the user's characteristic information (emissivity, color, luminosity, reflectance) of the skin surface. In this case, the manufacturer urges the user to install the second software, and if the user makes an upgrade to the second software from the first software, then it is possible to get the measurement result of the body temperature based on the new knowledge easily. Of course, the compensation formula/correction formula can be changed accordingly.

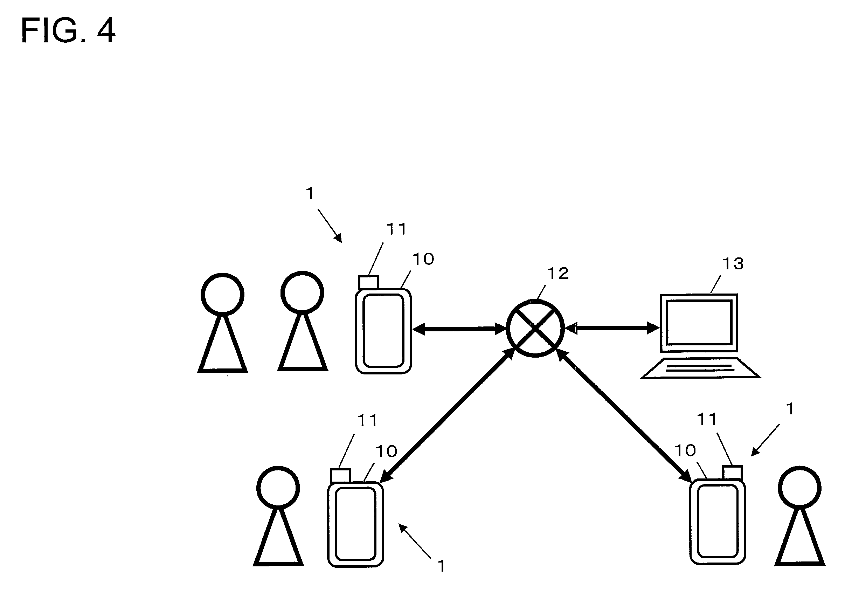

Also, as shown in Fig. 4, the terminal apparatus 10 of the body temperature measuring apparatus 1 according to the present invention is connected in a communicable to a server 13 through a network 12, such as the Internet, the server 13 is connected to several terminal apparatus 10 by wire or wireless through the network 12. A mutual communication between each terminal apparatus 10 and the server 13 is carried out with the software installed in each terminal apparatus 10. In this way, it is possible to construct a body temperature measuring system by connecting the body temperature measuring apparatus 1 to network 12.

The server 13 is a generally used computer, and serves as a database storing the user's body temperature in association with the user's account (identification data) and the user's attribute information (sex, position, or the like). The server 13 collects and stores body temperature information measured with each terminal apparatus 10 while making correlations with the user's account and attribute information. Therefore, the database becomes the big data.

Here, if the terminal apparatus 10 is connected in a communicable way to a biologically related information apparatus (pulsometer, sphygmomanometer, or the like) that measures biologically related information (pulse, blood pressure, blood oxygen levels, or the like) except the body temperature by wire or wireless, the terminal apparatus 10 or server 13 can store information regarding the user's body temperature and the user's biologically related information while making a association with the user's account. In this way, it is possible to manage the user's health information all at once.

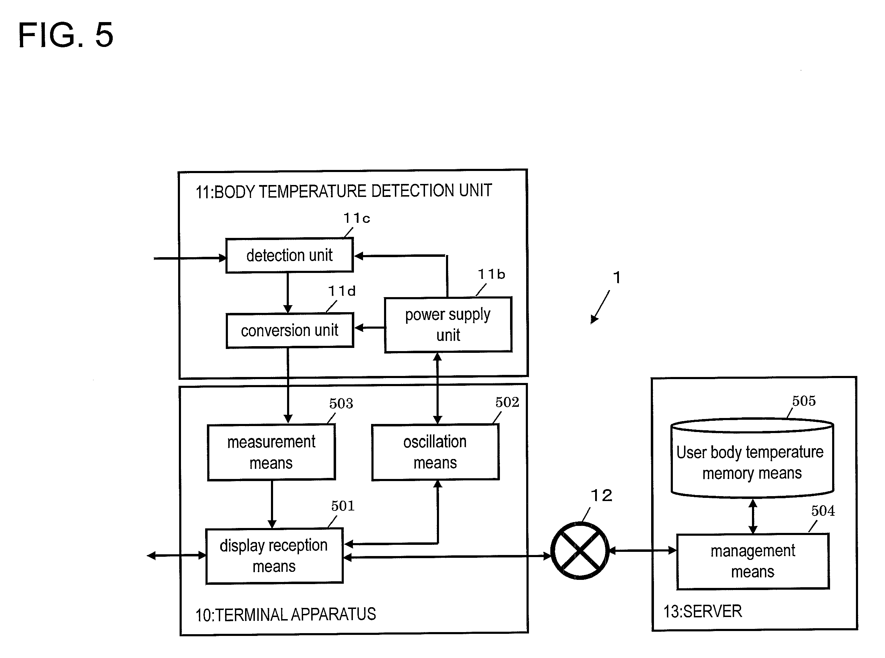

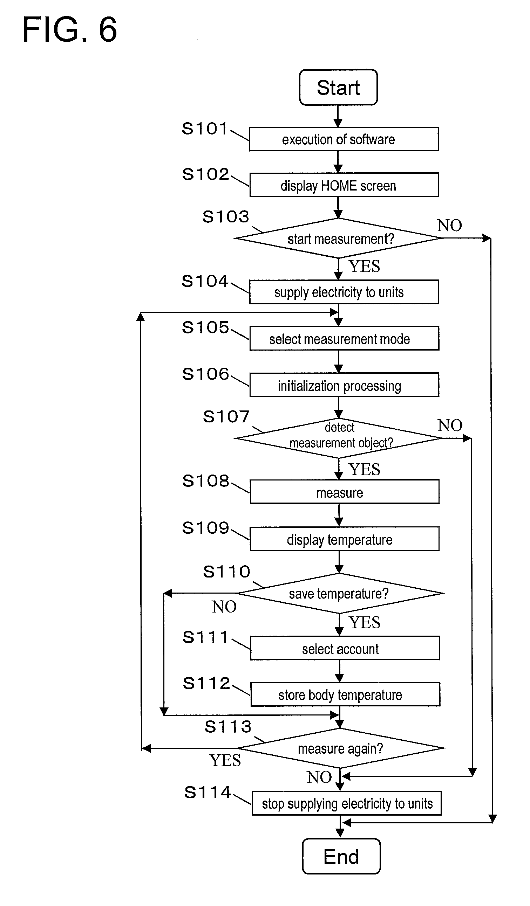

In accordance with Fig. 5, and Fig. 6, the structure and execution steps related to the embodiment of the present invention are explained.

### <Execution of the body temperature measurement>

When a user inserts the voice input/output terminal 11 a of the user's body temperature detection unit 11 to the voice input/output hole 10a of the user's portable terminal apparatus 10 (smartphone) with a touch panel, and turns on the power supply of the portable terminal apparatus 10, a display reception means 501 of the portable terminal apparatus 10 displays an initial screen on the touch panel, and receives a key operation of the user.

While the user watches the initial screen, for example, if the user inputs instructions for installing a specific application software for measuring body temperature, the display reception means 501 installs the software through the network. In this way, the portable terminal apparatus 10 and the body temperature detection unit 11 are constructed as a body temperature measuring apparatus 1.

Next, when the user inputs an execution instruction of the software, the display reception means 501 receives the execution instruction (Fig. 6, S101), and displays a body temperature measurement screen 700 (HOME screen) on the touch panel (Fig. 6, S102).

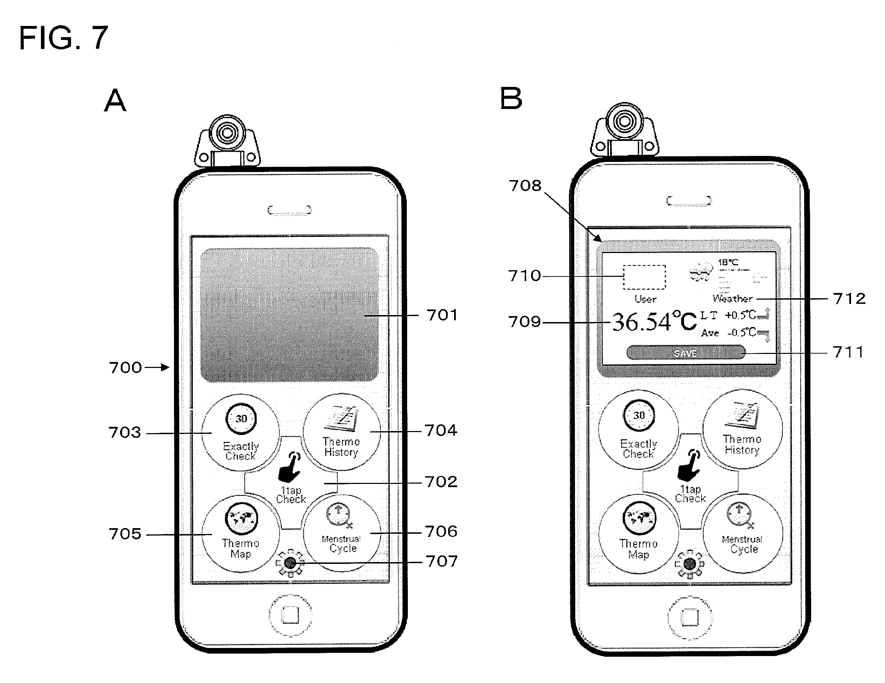

As shown in Fig. 7A, the body temperature measurement screen 700 displays a display screen 701, a speed check (1tapCheck) key 702, a 30 seconds check (ExactlyCheck) key 703, a body temperature history (ThermoHistory) key 704, a body temperature map (ThermoMap) key 705, a menstrual cycle (MenstrualCycle) key 706, and a setting key 707. The display screen 701 displays information, including precautions at the time of the measurement, descriptions during the measurement, and the result of the measurement.

Here, for example, when the user measures a body temperature and the user selects the speed check key 702, the display reception means 501 receives the selection of the speed check key 702 (Fig. 6, S103 YES), and notifies the selection to the oscillation means 502 of the software. Upon receipt of the notice, the oscillation means 502 oscillates a voice output signal to a voice output terminal (L/R). When a power supply unit 11b of the body temperature detection unit 11 receives the voice output signal, the power supply unit 11b converts these to electricity, and supplies the converted electricity to all units (a detection unit 11c, a conversion unit 11d) of the body temperature detection unit 11 (Fig. 6, S104).

Then, the display reception means 501 displays a measurement mode selection screen on the touch panel, and receives a selection of the measurement mode from the user (Fig. 6, S105). Here, the measurement mode means a measurement object and a measurement method. For example, in the first mode, a measurement object is the forehead of the user and the measurement method is a non-contact type. In the second mode, a measurement object is the forehead of the user and the measurement method is a contact type. In the third mode, a measurement object is the ear of the user and a measurement method is a contact type. In the fourth mode, a measurement object is an object other than the human body and the measurement method is a non-contact type.

For example, when the user selects the first mode, the display reception means 501 receives the first mode, and notifies the selection to a measurement means 503 of the software. Upon receipt of the notice, the measurement means 503 executes an initialization processing based on environmental temperature, the selected key, and the selected mode (Fig. 6, S106). For example, the initialization processing is the processing for selecting (adjusting) the compensation formula/ correction formula corresponding to the environmental temperature, type of key, and the mode.

For example, when a measurement object in the mode is set for the human body, the compensation formula's emissivity is selected as 0.98-0.99 with characteristic information such as color of the skin surface, luminosity, or the like. When a measurement object in the mode is set for an object, the compensation formula's emissivity is selected as 0.95. In addition, for example, when the speed check key 702 is selected, the compensation formula setting a measurement time to 5 seconds is selected. When the 30 seconds check key 703 is selected, the compensation formula setting a measurement time to 30 seconds is selected.

When the detection unit 11c detects a temperature detection signal corresponding to an environmental temperature, the conversion unit 11d converts the temperature detection signal to a voice input signal. And the measurement means 503 measures the environmental temperature based on the voice input signal, and executes the initialization processing. Next, the measurement means 503 determines whether or not the detection surface of the detection unit 11c detects a measurement object (Fig. 6, S107). Here, the method that the measurement means 503 determines does not have any limitations in particular. For example, when the initialization processing is completed, the measurement means 503 continues the reception of the voice input signal from the conversion unit 11d, and determines whether or not the voice input signal received is equal to the voice input signal corresponding to the environmental temperature. And when the voice input signal received is not equal to the voice input signal corresponding to the environmental temperature (when changed), the measurement means 503 determines the detection surface of the detection unit 11c detected a measurement object. When the voice input signal received is equal to the voice input signal corresponding to the environmental temperature, the measurement means 503 determines the detection surface of the detection unit 11c did not detect a measurement object.

For example, when the user touches his forehead with the detection surface of the detection unit 11c with a mirror, the detection surface of the detection unit 11c detects a measurement object. As a result of the determination in S107, when the detection surface of the detection unit 11c detects a measurement object (Fig. 6, S107 YES), the measurement means 503 receives a voice input signal within a predetermined measurement time from the point of time when the detection surface of the detection unit 11c detected a measurement object, applies the compensation formula/correction formula to the voice input signal, and measures the user's body temperature (e.g., "36.54 degrees") (Fig. 6, S108). In this way, even if the temperature detection signal is detected with a non-contact type, it is possible to calculate the user's body temperature with high precision. In addition, the above embodiment is the body temperature measurement of a non-contact type, but this goes the same even with a contact type. Also, the unit of body temperature can be either Celsius or Fahrenheit, and the user can change the setting.

When the speed check key 702 is selected, the measurement means 503 calculates the surface temperature 10 times for 5 seconds. When the 30 seconds check key 703 is selected, the measurement means 503 calculates the surface temperature 60 times for 30 seconds. If the measurement time increases, the accuracy of the body temperature measurement improves. The user can select the body temperature measurement with high accuracy, or the body temperature measurement quickly, depending on the intention of the user. In addition, a basal body temperature key is provided, and when the basal body temperature key is selected, the measurement means 503 can calculate the body temperature which is almost an actual value by calculating the surface temperature of a predetermined number in 1 minute to 5 minutes.

When the measurement means 503 finishes the body temperature measurement, it notifies the display reception means 501. Upon receipt of the notice, the display reception means 501 displays the result of the measurement on the display screen 701(708) of the body temperature measurement screen 700 (Fig. 6, S109). As shown in Fig. 7B, the display screen 708 of the body temperature measurement screen 700 displays the measured body temperature 709 ("36.54 degrees"), a user setting key 710, a storage (save) key 711, and weather information 712 including environmental temperature. In this way, the user can confirm the body temperature which he/she measured earlier. And the user setting key 710 which is displayed in Fig. 7B is displayed as a blank icon key because the user setting has not been complete.

When the user selects the save key 711 while looking at the display screen 708, the display reception means 501 receives the selection of the save key 711 (Fig. 6, S110 YES), and receives a selection of the user's account from the user (Fig. 6, S111). The selection of a user's account means that the user selects the previously set account, and the user can set a number of accounts. The following explanation will be when the user has not yet completed the account setting.

When the user has not yet completed the account setting, if the user selects the user setting key 710, then the display reception means 501 receives the selection of the user setting key. For example, it displays an account setting screen on the touch panel, and receives an input of a desired account (user ID) and a user's attribute information (attribute showing sex, positon showing location) from the user. Here, the account can be used with a user's name or even a nickname. In addition, the user's attribute information may be added, including sex, position (location), age, nationality, educational background, and work address.

When the user inputs "AAA" as an account, "woman" as an attribute, and "Kyoto" as a position while looking at the account setting screen, the display reception means 501 receives these information, accesses server 13 through network 12, and notifies a management means 504 of the server 13. Upon receipt of the notice, the management means 504 refers to a user body temperature table 800 which is memorized by a user body temperature memory means 505 of the server 13.

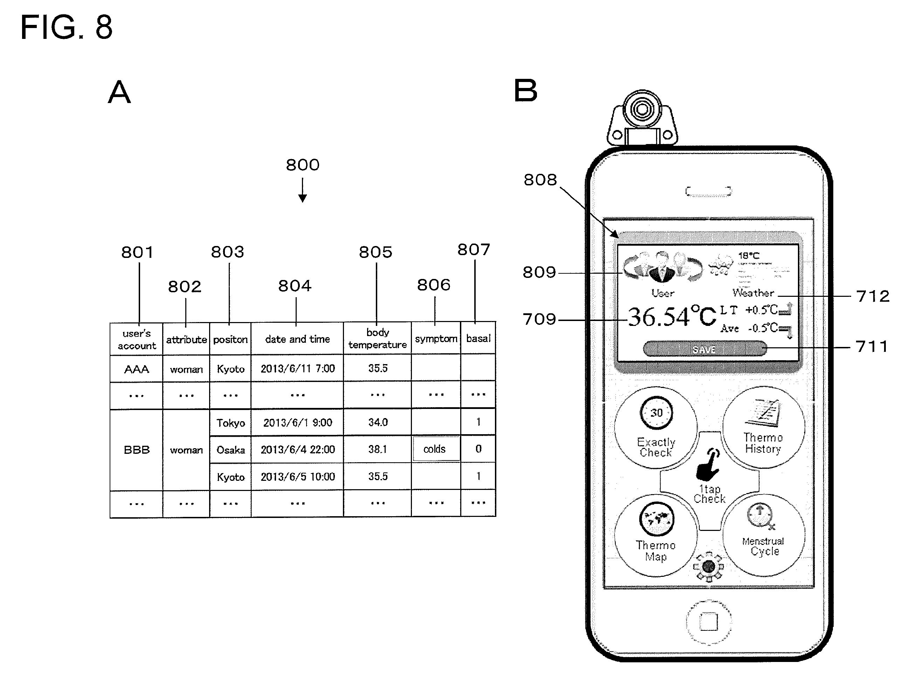

As shown in Fig. 8A, the user body temperature table 800 memorizes a user's account 801 while making a association with the user's attribute 802, the user's position 803, date and time 804 when the user measured body temperature, the user's body temperature 805 at the date and time 804, symptom 806 which indicates physical conditions such as colds, and a basal body temperature information 807 which shows whether it is a basal body temperature. This user body temperature table 800 becomes the database of the user account, the user's body temperature, and the user's attribute information.

The symptom 806 can be entered by the user as needed. Furthermore, the basal body temperature information 807 memorizes, for example, "1" which indicates the user's body temperature is a basal body temperature, or "0" which indicates the user's body temperature is not a basal body temperature, and this information 807 is configurable by the user as needed. In addition, a basal body temperature is a temperature measured in the rest state at which only minimum energy necessary for life support is used, and the basal body temperature is usually a temperature measured immediately when the user wakes up in the morning.

The management means 504 searches the account "AAA" which is entered by the user from the user body temperature table 800 and determines whether or not the input account exists in the user body temperature table 800. As a result of the determination, when the entered account exists in the user body temperature table 800, the management means 504 notifies the display reception means 501. Upon receipt of the notice, the display reception means 501 removes the entered account, and receives the input of another account which is different from the account by the user again. Also, as a result of the determination, when the account with input does not exist in the user body temperature table 800, the management means 504 stores the account with input "AAA", the attribute "woman", and the position "Kyoto" in the user body temperature table 800. In this way, the new account setting of the user is completed. And it becomes possible for the user to select his/her account.

When the user selects the account "AAA", the display reception means 501 receives the selection of the account (Fig. 6, S111), and notifies the management means 504. Upon receipt of the notice, the management means 504 refers to the user body temperature table 800, and stores the date and time "2013/6/11 7:00" of the body temperature measurement and the user's body temperature "36.54 degrees" in association with the selected account "AAA" (Fig. 6, S112). In this way, the user can easily store (accumulate) the measured body temperature in association with a predetermined account.

When the management means 504 stores the body temperature in association with the account "AAA", the management means 504 notifies this to the display reception means 501. Upon receipt of the notice, the display reception means 501 displays the result of the measurement on the display screen 808 of the body temperature measurement screen again. As shown in Fig. 8B, the display screen 808 of the body temperature measurement screen displays a user icon key 809 which indicates the account setting has been set. In this way, the user can confirm whether an account is selected at a glance.

Here, when the user selects the speed check key 702 or the like again while looking at the display screen 808, the display reception means 501 determines that the user will measure the body temperature again (Fig. 6, S113 YES), returns to S105, and receives the selection of the measurement mode by the user (Fig. 6, S105).

When the user selects any key of the body temperature history key 704, the body temperature map key 705, and the menstrual cycle key 706, the display reception means 501 notifies this to the management means 504. Upon receipt of the notice, the management means 504 displays the user's body temperature or the body temperature of the other account which has attribute information corresponding to the attribute information of the user's account.

### <Body temperature history display>

When the user selects the body temperature history key 704, the management means 504 refers to the user body temperature table 800, and acquires the user's body temperature 805, the symptom 806, and basal body temperature information 807 in the account 801 (e.g., "BBB") which has already been set. Here, for example, the management means 504 acquires the user's body temperature 805, the symptom 806, and basal body temperature information 807 in association with the date and time 804 from the current date up to the predetermined days (e.g., 7 days). And the management means 504 makes the display reception means 501 display a body temperature history screen 900 based on the referred user's body temperature 805, the symptom 806, the basal body temperature information 807, and the date and time 804.

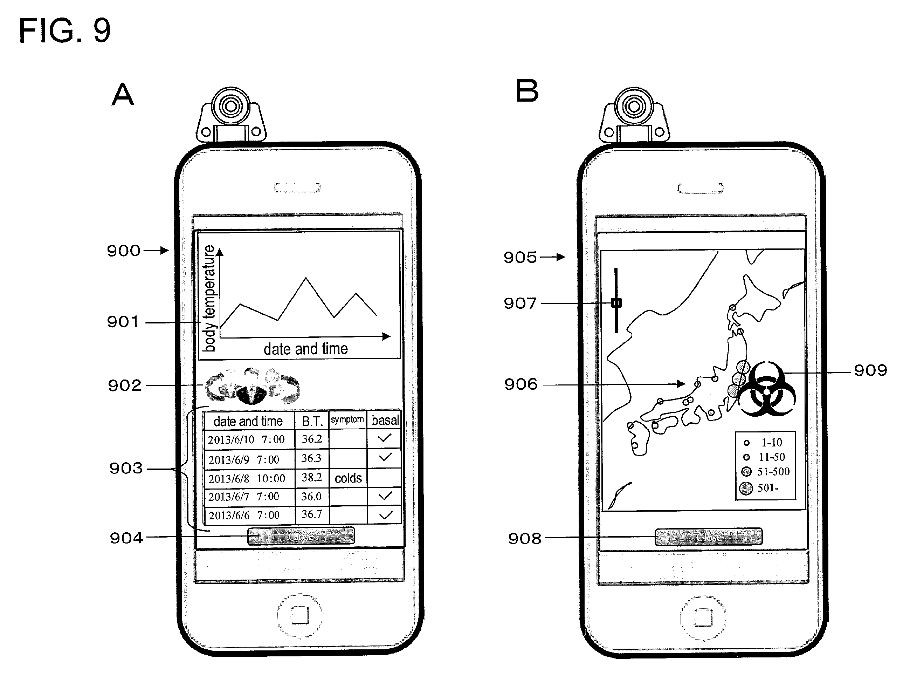

As shown in Fig. 9A, the body temperature history screen 900 displays a body temperature history graph 901, a user setting key 902 for making changes with the account settings, a table 903, and a close (Close) key 904. The body temperature history graph 901 is made based on the body temperature 805 and date and time 804 of account "BBB". The table 903 is made based on the date and time 804, the body temperature 805, symptom 806, and basal body temperature information 807 referred to from the account. As a result, the user can easily confirm the body temperature which he/she has measured before. In addition, the user can manage his/her health easily by confirming a temporal change of his/her body temperature.

Here, when the user selects the user setting key 902 and inputs an account ("AAA") of another, it is possible to switch from the body temperature history graph 901 and table 903 in the user's account "BBB" to a body temperature history graph and table in the account "AAA" of the other user. In addition, the table 903 is made up to receive input, such as shift checks which set a measured body temperature to a basal body temperature, symptoms, and memos. Here, for example, when a specific symptom (e.g., "colds") is added to the symptom 806, the management means 504 may display content and information of a hospital or clinic that exist around the position 803 by using the position 803 ("Kyoto") of the user.

Also, the management means 504 determines whether or not the subtracted value which is obtained to subtract the user's body temperature 805 from the environmental temperature exceeds a preset threshold value by using the user's body temperature 805 and the environmental temperature of weather information 712 in the position 803 of the user. And when the subtracted value exceeds the threshold value, the management means 504 may let the display reception means 501 display a message having possibilities for the user to suffer from heatstroke (heatstroke countermeasure alert). In addition, when the subtracted value is equal to or smaller than the threshold value, the management means 504 does nothing in particular.

Also, the management means 504 determines whether or not the user's body temperature 805 exceeds the fever body temperature by using the user's body temperature 805 and the fever body temperature (e.g., 38 degrees) which specifies a symptom of influenza. And when the user's body temperature 805 exceeds the fever body temperature, the management means 504 may display a message having possibilities for the user to have influenza. Furthermore, the management means 504 can determines whether or not the user has influenza with high accuracy by using the position of the user 803, the current date and time, infected zone of the influenza, and infection period of the epidemic for the influenza.

And when the body temperature 805 of a predetermined user exceeds the fever body temperature, the management means 504 determines whether or not the body temperature 805 of other users belonging to the position 803 of the user exceeds the fever body temperature. Furthermore, when the body temperature 805 of the other users exceeds the fever body temperature, the management means 504 counts the number of other users having the body temperature 805 which exceeds the fever body temperature. The management means 504 determines whether or not the number of other users who are counted exceeds a preset threshold value. When the number of the other user exceeds the threshold value, the management means 504 may let terminal apparatus 10 of the user in the position 803 display a message for an influenza epidemic.

### <Body temperature map display>

When the user selects the body temperature map key 705, the management means 504 refers to the user body temperature table 800 and refers to position 803 ("Kyoto") of the account 801 (e.g., "AAA") which is set by the user, the other position 803 (e.g., "Osaka", "Nara" in Japan) which is adjacent to position 803, the body temperature 805 of the other users (accounts) in association with the other position 803. And the management means 504 makes the display reception means 501 display a body temperature map screen 905 based on the referred information of the position 805 and the body temperature 805. As shown in Fig. 9B, the body temperature map screen 905 displays a body temperature map 906, a cursor key 907, and a closed key 908. The body temperature map 906 is made by placing the body temperature of the user and the other users at every position of the user and the other users. The cursor key 907 is for enlarging the body temperature map 906 to a wider area, or shrinking the body temperature map 906 to a narrower area.

The body temperature map 906 means what is illustrated for the body temperature of all users in every position to which the user belongs, or what is illustrated in the number of the users having the body temperature that is higher than the setting body temperature or a specific symptom for every position to which the user belongs. The body temperature map 906 may just display the body temperature and the number of the users to make it easier to view. The body temperature which is placed in the body temperature map 906 is displayed by the figures such as circles and the like. For example, the magnitude of the figures corresponds to that of the number of the users. In addition, for example, the high and low of the body temperature which is placed in the body temperature map 906 is displayed by setting a low temperature to blue and setting a high temperature to red. In Fig. 9B, the management means 504 counts the number of the users having a body temperature which exceeds the fever body temperature in a predetermined position (e.g., "Kyoto"), and places a circle of the size corresponding to the counted number in the position. Whenever the number of the users increases, the size of the circle enlarges progressively. In Fig. 9B, the ranges of the users from 1 to 10 are set in the circle of the smallest size, the ranges of the users from 11 to 50 are set in the circle of the second smallest size, the ranges of the users from 51 to 500 are set in the circle of the third smallest size, the ranges of the users more than 501 are set in the circle of the biggest size. In addition, the user's position which is displayed at the center of the body temperature map 906 is the position which the user inputs the position which is acquired by a predetermined GPS function of the portable terminal apparatus 10, or the designated position from the user. In this way, the user can confirm the body temperature of the other user in the position where the user is located or nearby.

For example, the management means 504 refers to the body temperature 805 and the position 803 in association with a specific symptom 806 such as heatstroke, infectious disease and the like, and displays the body temperature map which illustrates the body temperature 805 of the users having the specific symptom 806 in every position 803 of the users based on these referred information. And when the users having the specific symptom 806 is concentrated at a predetermined position (when the number of the users having the specific symptom 806 is large in a predetermined position), the management means 504 display an alert 909 indicating an infectious disease outbreak in the position 803 (pandemic alert). In this way, it is possible to grasp the body temperature and the position of the user and the other users related to a specific symptom with the body temperature map at a glance. Particularly, when the specific symptom 806 is influenza, it is possible to grasp the epidemic situation/spread of the influenza of each region at a glance (influenza infestation map).

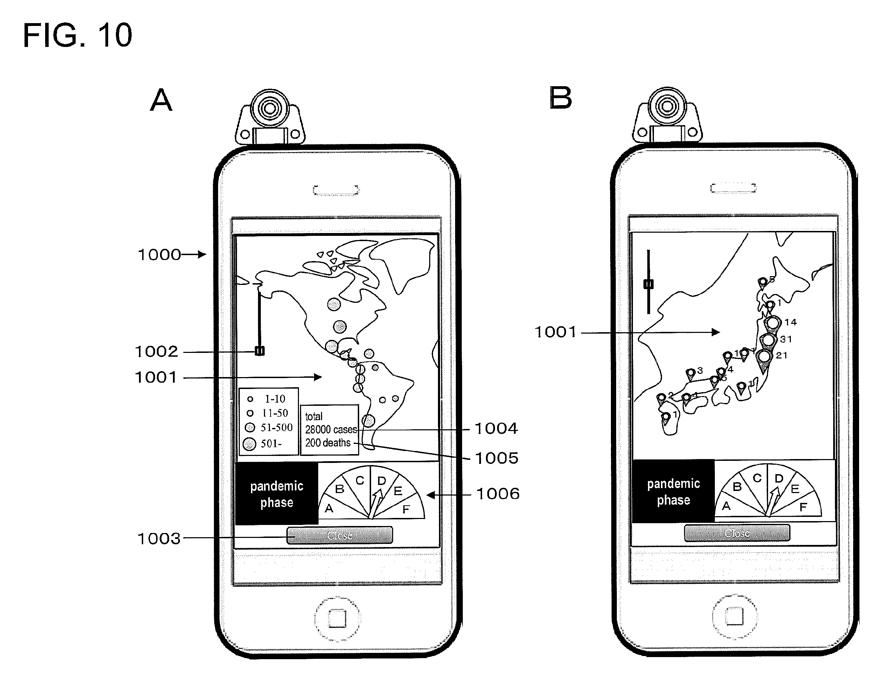

For example, when the user enlarges the body temperature map 906 to a wide area by operating the cursor key 907, the management means 504 re-collects the body temperature 805 of a position 803 enlarged in response to the key operation, and displays the body temperature map screen 1000 which is enlarged to a wide area. As shown in Fig. 10A, the body temperature map screen 1000 displays a body temperature map 1001, a cursor key 1002, and a closed key 1003. The body temperature map 1001 illustrates the body temperature of the user and the other users in a wide area (the whole world) for every position of each user. In this way, the user can confirm the body temperature (the mean) of him/her and the other users in every area, when the user enlarges or shrinks the displayed body temperature map 1001 by operating the cursor key 1002. In addition, as shown in Fig. 10A, the management means 504 counts the number of users having body temperature which exceeds the fever body temperature or the users having a specific symptom (influenza, a cold) among the users belonging to a predetermined position (e.g., "New York"), and places a circle of the size corresponding to the counted number to the position. And the management means 504 carries this out at all positions, and displays the total number 1004 of the counted number of the user as an infected person (cases) and the total number 1005 of the counted number of the user who is given "the dead" for the symptom as the dead (deaths). Furthermore, the management means 504 displays a phase 1006 (e.g., "D") of the pandemic alert corresponding to the total number of the infected person.

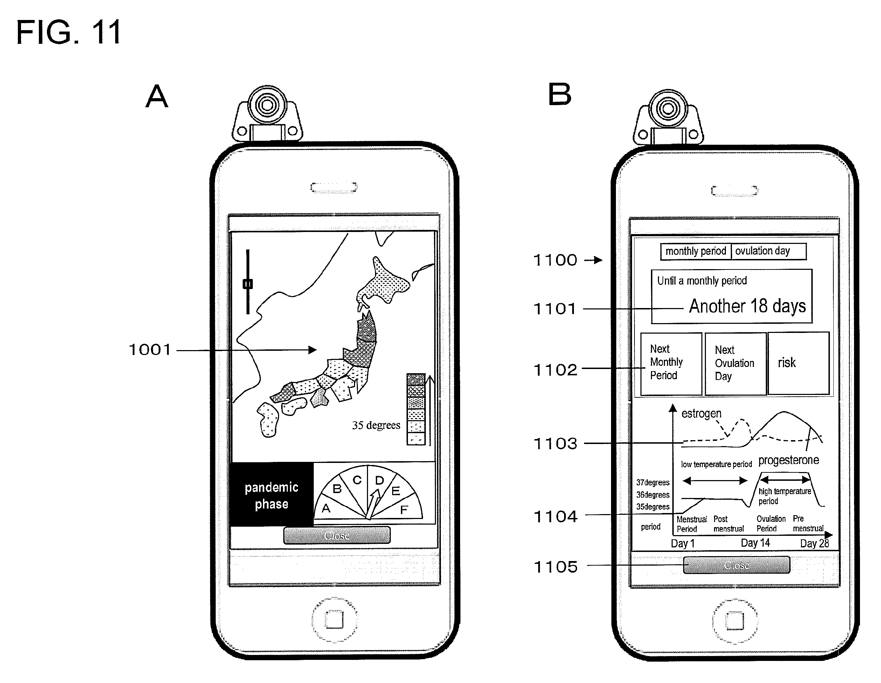

Also, as shown in Fig. 10B, the management means 504 may count the number of users having body temperature which exceeds the fever body temperature among users belonging to a predetermined position (e.g., "Kyoto"), and display the body temperature map 1001 by placing a reverse teardrop shape of the size corresponding to the counted number to the position. In this case, the counted number of the users is displayed as a reverse teardrop shape. As shown in Fig. 11A, the management means 504 may collect the body temperature of the user and the other user belonging to a predetermined area in the map, calculate the mean of the collected body temperature of the users, and display a body temperature map 1001 which separates the calculated mean by color depending on high and low. In this case, it is possible to understand the high and low of the body temperature in every area at a glance. In addition, for example, the range of the body temperature map is set to the range displaying the details of the current position 803 of the users as default, and can be set to the wide area range by a user operating the key.

### <Menstruation cycle display>

When the user selects the menstrual cycle key 706, the management means 504 refers the date and time 804, the body temperature 805, the basal body temperature information 807 in association with the current account 801 ("BBB") setting in the user body temperature table 800. And the management means 504 displays a menstruation cycle screen 1100 based on the date and time 804 and the body temperature 805 corresponding to a basal body temperature. As shown in Fig. 11B, the menstruation cycle screen 1100 displays residual date 1101, menstruation cycle information 1102, a menstruation cycle graph 1103, a secretion graph 1104 for hormone, and a closed key 1105. The residual date 1101 is the remaining days from the current date and time to the monthly period. The menstruation cycle information 1102 is the monthly period and the ovulation day that is predicted from the body temperature 805 and the date and time 804 of the user who is a woman. The menstruation cycle graph 1103 is made based on the body temperature 805 and the date and time 804 of the user who is a woman. The secretion graph 1104 for hormone is made in response to the menstruation cycle graph 1103.

And a high temperature period and a low temperature period characterized in a menstruation cycle are displayed to the menstruation cycle graph 1103, and a menstrual period and postmenstrual are displayed to the low temperature period, while an ovulation period and premenstrual are displayed to the high temperature period. In this way, the user can confirm her menstruation cycle easily, and grasp what time is a monthly period or an ovulation cycle easily. Here, when the management means 504 displays the menstruation cycle screen of the user, determines whether or not the current date and time are included in a physical condition related time related to a physical condition of the menstruation cycle of the user. And when the current date and time are included in the physical condition related time, the management means 504 may display information to improve the physical condition of the user in response to the physical condition related time in the menstruation cycle screen. In this way, it is possible to inform information to improve the health care of the user in response to the menstruation cycle of the user.

For example, one week from the postmenstrual to the ovulation period in the menstruation cycle is called a "lean phase", and a metabolism of the user improves by a decrease in estrogen (ovarian follicle hormone). Therefore, the management means 504 determines whether or not the current date and time are included in the "lean phase" of the menstruation cycle of the user. When the current date and time are included in the "lean phase", the management means 504 displays contents and information that are related to the diet on the menstruation cycle screen. In this way, it is possible to enhance the diet effect of the user by displaying the diet related information to the user effectively.

Also, one week from the ovulation period of the menstruation cycle is called a "detox phase", water and energy are easy to be stored to the body of the woman by increase in progesterone, and skin trouble such as excessive secretion of the sebum, acne, or the like are easy to occur. Therefore, the management means 504 determines whether or not the current date and time are included in the "detox phase" of the menstruation cycle of the user. When the current date and time are included in the "detox phase", the management means 504 displays contents and information that are related to skin troubles and beautiful skin in the menstruation cycle screen. In this way, it is possible to enhance the beautiful skin effect of the user by displaying the skin trouble related information to the user effectively.

And one week before menstruation during the menstruation cycle is called an "unstable phase" where disorders such as edema, lower abdominal pain, tiredness and the like are easy to occur to the body of a woman, and it is easy to become mentally unstable. Therefore, the management means 504 determines whether or not the current date and time are included in the "unstable phase" of the menstruation cycle of the user. When the current date and time are included in the "unstable phase", the management means 504 displays contents and information that are related to Chinese medicine and supplements in the menstruation cycle screen. In this way, it is possible to promote physical condition management of the user by displaying information related to improving the physical condition to the user effectively.

### <Setting>

When the user selects the setting key 707, the display reception means 501 receives the selection of the setting key 707, and the management means 504 receives various settings. For example, the settings are a modification or a deletion of the account, the attribute, the position, the date and time, the body temperature, the symptom, and basal body temperature information in the user temperature table 800. In addition, the settings include an alarm setting to generate alarms when the current date and time is a specific date and time.

### <End of body temperature measurement>

In S113, when the user selects a predetermined cancel key without doing any key operations (Fig. 6, S113 NO), the display reception means 501 receives the selection of the cancel key, and notifies the oscillation means 502. Upon receipt of the notice, the oscillation means 502 stops the oscillation of the voice output signal, and stops the power supply (Fig. 6, S114). In S107, after the elapsed time from the determination start point exceeds a predetermined time, when the voice input signal received is equal with the voice input signal corresponding to the environmental temperature, the measurement means 503 determines the detection surface of the detection unit 11c does not detect a measurement object (Fig. 6, S107 NO). And the steps goes back to S114, the oscillation means 502 stops the power supply (Fig. 6, S114). In this way, the execution of the specific applications software is finished. The display reception means 501 displays the initial screen again, and receives different key operation from the user.

In addition, the present invention can be combined with a biologically related information apparatus which is connected in a communicable way to the terminal apparatus 10 by a close distance wireless communication technology such as Bluetooth (registered trademark) and the like. For example, the management means 504 stores the user's body temperature and the biologically related information in accordance with the account of the user, analyzes these, and displays these. Therefore, it is possible realize physical condition management and detect serious diseases of the user in its early stages.

Also, in the present invention, the management means 504 of the server 13 can correlate a number of the users having equal attribute information (e.g., a symptom). For example, the management means 504 specifies the account of the other users having the attribute information which is equal to the attribute information (e.g., a cold) of the set account of the user, and introduce the other users of the specified account to the user of the set account. In this way, it is possible to improve mutual interaction among the user and the other users experiencing the same health issues. In addition, the management means 504 may have the set user interact with the other user specified on the display screen or social network by connecting the account of the user body temperature table 800 to a social network account such as Facebook, Twitter, or the like.

Especially, social networks has big data to connect the users of the world, and the users of the world can consult the same health condition mutually and report an improvement of the health condition by corresponding to the user body temperature table 800 (the big data) of the present invention to the big data of the social network. In this way, it is possible to enhance health awareness of users in the world.

In addition, the management means 504 may rank the health condition of users by using the presence/absence of the symptom of the users in the user body temperature table 800, and execute a ranking display which the users can browse. And the management means 504 may use the symptom of the user body temperature table 800, and notify the body temperature of users to the terminal apparatus of a doctor and a relative who are in a distant place by using the position 803 of the account 801 of the users with a serious symptom. And the management means 504 may analyze the preference of the users based on the user's body temperature and the attribute information (e.g., the symptom 806) of the users in the set account in the user body temperature table 800, acquire information related to the preference of the user in the other terminal apparatus, and display the acquired information to the users (recommendation). In addition, the management means 504 may acquire advertisement sites related to the preference of the users in the other terminal apparatus, and display the acquired information to the users (advertisement delivery).

### INDUSTRIAL APPLICABILITY

As described above, a body temperature measuring apparatus, a body temperature measuring system, and a body temperature measuring method are useful as tools of the field related to the body temperature of the user such as in the medical field, hygienic field, and the like. It is effective as a body temperature measuring apparatus, a body temperature measuring system and a body temperature measuring method which can measure the body temperature with high accuracy, a non-battery type, and a non-contact type, and realize improvement in accuracy in measuring the body temperature with software version upgrades easily.

### REFERENCE SIGNS LIST

- 1: Body temperature measuring apparatus
- 10: Terminal apparatus
- 11: Body temperature detection unit
- 12: Network
- 13: Server
- 501: Display reception means
- 502: Oscillation means
- 503: Measurement means
- 504: Management means
- 505: User body temperature memory means

## Claims

1. A body temperature measuring apparatus comprised of a terminal apparatus and a body temperature detection unit that are connected through a voice input/output hole and a voice input/output terminal, the body temperature detection unit comprising:
a power supply unit for converting a voice output signal from the terminal apparatus into electricity and supplying the converted electricity to its respective units of the body temperature detection unit;
a detection unit for detecting infrared energy as a temperature detection signal from the user's skin surface separated from a detection surface to a predetermined measurement distance;
a conversion unit for converting the temperature detection signal into a voice input signal and transmitting the converted voice input signal to the terminal apparatus; and
the terminal apparatus comprising:
a oscillation means for oscillating a voice output signal to the power supply unit of the body temperature detection unit; and
a measurement means for calculating surface temperature of the skin surface based on the voice input signal received form the conversion unit and an emissivity of the user's skin surface, and estimating the user's body temperature corresponding to the surface temperature of which the variation is small based on the temporal variation of the surface temperature calculated within a predetermined measurement time.

2. A body temperature measuring system comprised of the body temperature measuring apparatus according to claim 1, connected in a communicable way to a server through a network, a terminal apparatus comprising:
a display reception means for displaying the user's estimated body temperature and receiving a selection of a account from the user; and
a server comprising:
a management means for storing the user's body temperature in association with the user's account received from the terminal apparatus in a user body temperature memory means and making the terminal apparatus display the user's body temperature in association with the user's account and the user's body temperature estimated before.

3. The body temperature measuring system according to claim 2, wherein
the management means stores attribute information including user's sex, a positon showing user's location, and date and time when the user's body temperature is estimated in association with the user's account and the user's body temperature in the user body temperature memory means, and makes the terminal apparatus display any one of the body temperature history graph made based on the user's body temperature and the date and time, a body temperature map made by placing the body temperature of the user and the other users at every position of the user and the other users, and a menstruation cycle graph made based on the body temperature and the date and time of the user who is a woman.

4. The body temperature measuring system according to claim 3 wherein
the management means determines whether or not the current date and time included in a physical condition related time is related to a physical condition of the menstruation cycle of the user in displaying the menstruation cycle graph, when the current date and time are included in the physical condition related time, while the management means displays information to improve the physical condition of the user in response to the physical condition related time in the menstruation cycle graph.

5. A body temperature measuring method of a body temperature measuring apparatus comprised of a terminal apparatus and a body temperature detection unit that are connected through a voice input/output hole and a voice input/output terminal, comprising steps of:
oscillating a voice output signal form the terminal apparatus to the body temperature detection unit;
converting the voice output signal from the terminal apparatus into electricity and supplying the converted electricity to its respective units of the body temperature detection unit;
detecting infrared energy as a temperature detection signal from the user's skin surface separated from a detection surface to a predetermined measurement distance;
converting the temperature detection signal into a voice input signal and transmitting the converted voice input signal to the terminal apparatus; and
calculating surface temperature of the skin surface based on the voice input signal received form the body temperature detection unit and an emissivity of the user's skin surface, and estimating the user's body temperature corresponding to the surface temperature of which the variation is small based on the temporal variation of the surface temperature calculated within a predetermined measurement time.
